# EUROPÄISCHE PATENTSCHRIFT

(11) **EP 0 695 284 B1**
(45) Veröffentlichungstag und Bekanntmachung des Hinweises auf die Patenterteilung: **18.11.1998**
(21) Anmeldenummer: 94914408.3
(22) Anmeldetag: 19.04.1994
(51) Int. Cl.: C07C 29/32, C07C 29/44, C07C 31/125, C07C 31/135

(54) **KONTINUIERLICHER PROZESS ZUR HERSTELLUNG VON TERTIÄREN ALKOHOLEN DURCH RADIKALISCHE ADDITIONSREAKTION VON SEKUNDÄREN ALKOHOLEN AN ALKENE**
CONTINUOUS PRODUCTION PROCESS OF TERTIARY ALCOHOLS BY RADICAL ADDITION OF SECONDARY ALCOHOLS TO ALKENES
PROCEDE DE PRODUCTION EN CONTINU D'ALCOOLS TERTIAIRES PAR ADDITION RADICALAIRE D'ALCOOLS SECONDAIRES SUR DES ALCENES

(30) Priorität: 20.04.1993 DE 4312815
(43) Veröffentlichungstag der Anmeldung: 07.02.1996
(73) Patentinhaber: Peroxid-Chemie GmbH, 82049 Pullach (DE)
(72) Erfinder: GNANN, Michael, D-82049 Gro hesselohe (DE); ECKERT, Maria, D-82547 Eurasburg (DE); RIETH, Robert +di, XX (DE)
(74) Vertreter: Weiss, Wolfgang, Dipl.-Chem. Dr.
(86) Internationale Anmeldenummer: EP9401214
(87) Internationale Veröffentlichungsnummer: WO9424078

(56) Entgegenhaltungen:
- GB-A- 708 254
- US-A- 3 255 260
- 'METHODEN DER ORGANISCHEN CHEMIE TEIL3, BAND 6/1B' 1984 , G. THIEME VERLAG , STUTTGART,DE in der Anmeldung erwähnt siehe Seite 655

## Beschreibung

Die vorliegende Erfindung betrifft ein neues Verfahren zur Herstellung von tertiären Alkoholen durch radikalische Addition von sekundären Alkoholen an Kohlenstoff-Kohlenstoff-Doppelbindungssysteme.

Die Addition von sekundären Alkoholen an Alkene unter Bildung von tertiären Alkoholen ist prinzipiell in der Technik bekannt.

US-A-3,352,929 beschreibt die Herstellung von Kondensationsprodukten aus Isopropanol und Acetylenverbindungen. Dieses Verfahren kann in zwei Stufen durchgeführt werden, wobei die erste Stufe die Reaktion einer Acetylenverbindung mit Isopropanol zu einem Alkenol umfaßt, und die zweite Stufe die Addition eines weiteren Isopropanols unter Bildung eines gesättigten tertiären (mehrwertigen) Alkohols umfaßt. Diese Reaktion kann in Gegenwart von organischen Peroxiden als Katalysator durchgeführt werden. Gemäß US-A-3,352,929 (Spalte 5, Zeilen 43-51) ist für die erste Stufe der Reaktion, nämlich die Addition eines sekundären Alkohols an eine Kohlenstoff-Kohlenstoff-Dreifachbindung, nur eine geringe Reaktionsdauer von z.B. 5 Minuten erforderlich, während für die zweite Stufe, nämlich die Addition eines weiteren Moleküls Isopropanol an das eine Kohlenstoff-Kohlenstoff-Doppelbindung aufweisende Additionsprodukt der ersten Reaktionsstufe, eine erheblich längere Reaktionsdauer von etwa 2 bis 5 Stunden erforderlich ist.

Weiterhin ist aus Methoden der organischen Chemie (Houben-Weyl), Band VI/1b, Georg Thieme Verlag Stuttgart, New York, 1984, Seite 654 ff) bekannt, daß durch Addition von Alkoholen (z.B. sekundären Alkoholen wie etwa Isopropanol, Isobutanol) an Alkene tertiäre Alkohole erhalten werden können. Die Reaktion erfolgt durch Erhitzen des Olefins in überschüssigem Alkohol (in einem Molverhältnis von Olefin zu Alkohol von 1:10-50) unter Zusatz eines Dialkylperoxids als Initiator (10 bis 20 Molprozent bezüglich des Olefins) auf 110 bis 135°C für eine Zeitdauer von 35 bis 40 Stunden. In dieser Literaturstelle ist beschrieben, daß bei dieser Reaktion durch Telomerisation überwiegend höhere Alkohole erhalten werden, während das monomere Additionsprodukt nur in einer relativ geringen Ausbeute entsteht.

Auch US-A-3,255,260 offenbart ein Verfahren zur Herstellung oligomerer Additionsprodukte durch radikalische Addition von sekundären Alkoholen an Alkene. Monomere Additionsprodukte werden lediglich als unerwünschte Nebenprodukte in geringer Ausbeute erhalten. GB-A-708 254 betrifft ein Verfahren zur Herstellung oligomerer Additionsprodukte durch radikalische Addition von Alkohol an ein Alken, wobei in einem diskontinuierlichen Verfahren die Reaktionspartner mindestens 4 Stunden in Kontakt gehalten werden. Bei diesem Verfahren werden überwiegend höhere Additionsprodukte gebildet, während das monomere Additionsprodukt nur in sehr geringen Mengen anfällt.

Ein schwerwiegender Nachteil der oben beschriebenen Reaktionen besteht somit darin, daß durch die hohe Kontaktzeit der Reaktanden von mehreren Stunden erhebliche Anteile an telomeren bzw. polymeren Nebenprodukten im Reaktionsgemisch entstehen. Ein weiterer Nachteil der Verfahren des Standes der Technik ist die durch die lange Reaktionsdauer bedingte geringe Raum/Zeit-Ausbeute an monomerem Additionsprodukt.

Die der vorliegenden Erfindung zugrundeliegende Aufgabe bestand somit darin, ein Verfahren zur Berstellung von tertiären Alkoholen durch Addition von sekundären Alkoholen an Alkene bereitzustellen, bei dem die oben genannten Nachteile völlig oder zumindest weitgehend vermieden werden können.

Die erfindungsgemäße Aufgabe wird gelöst durch ein Verfahren zur Berstellung von tertiären Alkoholen durch radikalische Addition von sekundären Alkoholen an Alkene, welches dadurch gekennzeichnet ist, daß man die Reaktion als koninuierlichen Prozeß in Gegenwart eines organischen Peroxids als radikalischem Initiator, einem Verhältnis von 3 bis 50 mol sekundärer Alkohol pro mol eingesetztem Alken und mit einer mittleren Kontaktzeit des Reaktionsgemisches von bis zu maximal 1 Stunde durchführt.

Überraschenderweise wurde festgestellt, daß durch die kontinuierliche Verfahrensführung gemäß vorliegender Erfindung bereits bei einer Kontaktzeit des Reaktionsgemisches von bis zu maximal 1 Stunde, vorzugsweise bis zu maximal 30 Minuten, besonders bevorzugt bis zu maximal 20 Minuten ein monomeres Additionsprodukt in hoher Raum/Zeit-Ausbeute und mit einem geringeren Anteil an polymeren oder/und telomeren Nebenprodukten erhalten wird.

Die Herstellung von tertiären Alkoholen durch eine radikalische Additionsreaktion von sekundären Alkoholen an Kohlenstoff-Kohlenstoff-Doppelbindungssysteme findet nach dem folgenden Reaktionsschema statt: 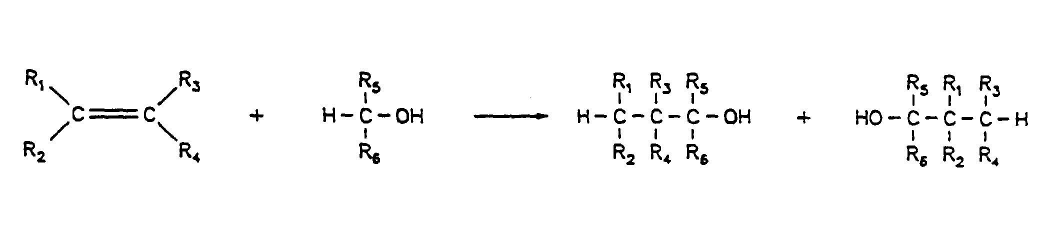 wobei R₁, R₂, R₃ und R₄ Wasserstoff oder gegebenenfalls substituierte Alkyl- oder Arylreste bedeuten und R₅ und R₆ gegebenenfalls substituierte Alkyl- oder Arylreste bedeuten.

Das erfindungsgemäße Verfahren besitzt einen breiten Anwendungsbereich, da es vorteilhaft mit einer großen Zahl verschiedener Alkene und sekundärer Alkohole durchgeführt werden kann. Als Alkene verwendet man vorzugsweise Verbindungen mit 2 bis 20 C-Atomen, besonders bevorzugt 4 bis 16 C-Atomen, die gegebenenfalls Substituenten tragen können, welche die Reaktion nicht beeinträchtigen (z.B. OH-Gruppen, O-Alkylgruppen, aromatische Gruppen). Spezifische Beispiele für besonders bevorzugte Alkene sind 2-Methyl-3-buten-2-ol, 3-Methyl-3-buten-1-ol, 1-Tetradecen, 1-Dodecen, Cyclohexen und α-Methylstyrol. Das am meisten bevorzugte Alken ist 2-Methyl-3-buten-2-ol.

Als sekundärer Alkohol werden bei dem erfindungsgemäßen Verfahren Substanzen mit vorzugsweise 3 bis 14 C-Atomen, besonders bevorzugt 3 bis 10 C-Atomen verwendet. Erfindungsgemäß wird der sekundäre Alkohol in einem Überschuß von 3 bis 50 Mol, besonders bevorzugt von 3 bis 20 Mol Alkohol pro Mol eingesetztem Alken verwendet. Spezifische Beispiele für bevorzugte sekundäre Alkohole sind 2-Propanol (Isopropanol), 2-Butanol, 2-Octanol, Cyclohexanol, 3,3-Dimethylbutan-2-ol, 2,6-Dimethylheptan-4-ol und 1-Phenylethanol. Der am meisten bevorzugte sekundäre Alkohol ist Isopropanol.

Besonders bevorzugt wird das erfindungsgemäße Verfahren daher zur Addition von Isopropanol an 2-Methyl-3-buten-2-ol verwendet, wobei als Additionsprodukt 2,5-Dimethylhexan-2,5-diol entsteht.

Das erfindungsgemäße Verfahren wird weiterhin in Gegenwart eines organischen Peroxids als radikalischem Initiator durchgeführt. Bevorzugte Beispiele für organische Peroxide sind Peroxycarbonsäureester (z.B. tert.-Butylperoxypivalat, tert.-Amylperoxypivalat), Dialkylperoxide (z.B. Di-tert.-Butylperoxid, Di-tert.-Amylperoxid) und Peroxyketale. Die Menge des radikalischen Initiators beträgt vorzugsweise 1 bis 50 Molprozent, besonders bevorzugt 5 bis 30 Molprozent bezüglich des eingesetzten Alkens.

Die Temperatur bei dem erfindungsgemäßen Verfahren beträgt vorzugsweise je nach eingesetztem radikalischen Initiator zwischen 50 und 240°C, besonders bevorzugt zwischen 80 und 200°C. Bei Verwendung von Di-tert.-Butylperoxid als Initiator beträgt die Temperatur vorzugsweise zwischen 150 und 200°C. Je nach eingesetztem Alken kann die Reaktion unter Normaldruck oder unter erhöhtem Druck durchgeführt werden. Es ist jedoch im allgemeinen bevorzugt, die Reaktion unter erhöhtem Druck und in einem Druck-Rohrreaktor durchzuführen. Bei einer derartigen Verfahrensführung kann der Druck beispielsweise in einem Bereich von 5 bis 100 bar (500 kPa bis 10000 kPa) liegen.

Weiterhin ist es bei dem erfindungsgemäßen Verfahren bevorzugt, nach Beendigung der Reaktion überschüssigen sekundären Alkohol aus dem Produktgemisch kontinuierlich zu entfernen (z.B. durch Destillation) und in das Reaktionsgemisch zurückzuführen.

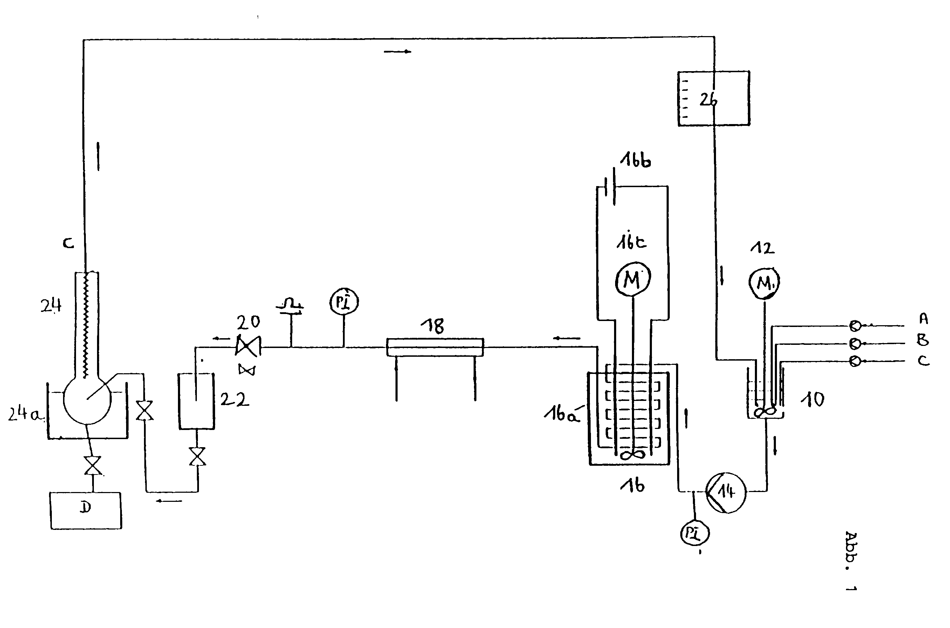

In Abbildung 1 ist ein Diagramm für eine besonders bevorzugte Ausführungsform des erfindungsgemäßen Verfahrens zu erkennen. Dabei werden die Reaktionspartner, nämlich das Alken (A), der radikalische Initiator (B) und der sekundäre Alkohol (C) über drei getrennte Leitungen in ein Mischgefäß 10 geleitet, das mit einem Rührer 12 versehen ist. Das Reaktionsgemisch wird aus dem Mischgefäß 10 über eine Pumpe 14 in einen Rohrreaktor 16 geleitet, der sich in einem Heizbad 16a mit Heizstab 16b und Rührer 16c befindet. Die Kontaktzeit des Reaktionsgemisches im Rohrreaktor beträgt dabei vorzugsweise 10 bis 20 Minuten. Dann wird das Gemisch durch einen Kühler 18 und ein Druckentspannungsventil 20 zu einem Aufnahmegefäß 22 geleitet. Vom Aufnahmegefäß 22 wird das Reaktionsgemisch zu einer Destillationseinheit 24 geleitet, die mit einem Heizbad 24a ausgestattet ist. Dort kann das Reaktionsprodukt (D) nach dem Abdestillieren von nicht umgesetztem sekundärem Alkohol (C) aus dem Destillationsrückstand isoliert werden. Der abdestillierte sekundäre Alkohol (C) wird in einem Destillatgefäß 26 gesammelt und von dort aus wieder dem Mischgefäß 10 zudosiert.

Die Erfindung wird weiterhin durch die folgenden Beispiele veranschaulicht.

### BEISPIEL 1

2-Methyl-3-buten-2-ol, 2-Propanol und Di-tert.-butylperoxid werden in einem Molverhältnis von 1:10:0,2 in ein mit einem Rührer versehenes Gefäß geleitet und dort vermischt. Dieses Reaktionsgemisch wird bei einem Druck von 3000 kPa kontinuierlich durch die Rohrschlangen eines 1000 ml Rohrreaktors gepumpt. Dabei befindet sich der Rohrreaktor in einem auf 180°C geheizten Wärmebad. Die Durchflußrate durch den Rohrreaktor wird so eingestellt, daß die Kontaktzeit des Reaktionsgemisches im Reaktor ca. 15 Minuten beträgt (3,8 l/h). Das eingesetzte Olefin hat sich in dieser Zeit vollständig umgesetzt.

Nach Verlassen des Reaktors wird das Reaktionsgemisch abgekühlt und über ein Druckreduzierventil in einem Aufnahmebehälter gesammelt, von dem es in eine Destillationsvorrichtung geleitet werden kann. Bei der anschließenden Destillation wird das überschüssige 2-Propanol entfernt und kontinuierlich in den Prozeß rückgeführt. Das Additionsprodukt 2,5-Dimethylhexan-2,5-diol befindet sich im Destillationsrückstand. Es werden 380 g Produkt/h gewonnen (Reinheit > 97 GC-Fl.-%).

### BEISPIEL 2

1-Tetradecen, 2-Propanol und Di-tert.-butylperoxid werden in einem Molverhältnis von 1 : 15 : 0,1, wie in Beispiel 1 beschrieben, bei einem Druck von 3000 kPa zur Reaktion gebracht. Die Kontaktzeit des Reaktionsgemisches im auf 175°C beheizten Reaktor beträgt ca. 20 Minuten. 1-Tetradecen hat sich nach dieser Zeit vollständig umgesetzt. Das Additionsprodukt, 2-Methyl-2-hexadecanol, wird mit einer Selektivität von 65 % gebildet und, wie in Beispiel 1 beschrieben, isoliert.

### BEISPIEL 3

2-Methyl-3-buten-2-ol, Cyclohexanol und Di-tert.-butylperoxid werden bei 180°C in einem Molverhältnis von 1 : 15 : 0,25 bei einem Druck von 2500 kPa, wie in Beispiel 1 beschrieben, zur Reaktion gebracht. Nach einer Reaktionszeit von 15 Minuten hat sich das eingesetzte 2-Methyl-3-buten-2-ol bereits vollständig umgesetzt. Aus dem Destillationsrückstand wird das Additionsprodukt, wie in Beispiel 1 beschrieben, mit einer Selektivität von 85 % isoliert.

### BEISPIEL 4

2-Methyl-3-buten-2-ol, 2-Propanol und tert.-Butylperoxypivalat (75 %ige Lösung in Aliphaten) werden in einem Molverhältnis von 1 : 15 : 0,3 bei 90°C und einem Druck von 500 kPa, wie in Beispiel 1 beschrieben, zur Reaktion gebracht. Nach 20 Minuten Kontaktzeit des Reaktionsgemisches im Rohrreaktor hat sich das eingesetzte Olefin zu 95 % umgesetzt. 2,5-Dimethylhexan-2,5-diol wird mit 76 % Selektivität gebildet.

## Patentansprüche

1. Verfahren zur Herstellung von monomeren tertiären Alkohol-Additionsprodukten durch radikalische Addition eines sekundären Alkohols an ein Alken,
**dadurch gekennzeichnet,**
daß man die Reaktion als kontinuierlichen Prozess in Gegenwart eines organischen Peroxids als radikalischem Initiator, einem Verhältnis von 3 bis 50 mol sekundärer Alkohol pro mol eingesetztem Alken und mit einer mittleren Kontaktzeit des Reaktionsgemisches von bis zu maximal 1 Stunde durchführt.

2. Verfahren nach Anspruch 1,
**dadurch gekennzeichnet,**
daß man die Reaktion mit einer mittleren Kontaktzeit des Reaktionsgemisches von bis zu maximal 30 Minuten durchführt.

3. Verfahren nach Anspruch 1 oder 2,
**dadurch gekennzeichnet,**
daß man ein Alken mit 2 bis 20 C-Atomen verwendet.

4. Verfahren nach einem der Ansprüche 1 bis 3,
**dadurch gekennzeichnet,**
daß man einen sekundären Alkohol mit 3 bis 14 C-Atomen verwendet.

5. Verfahren nach einem der Ansprüche 1 bis 4,
**dadurch gekennzeichnet,**
daß man als radikalischen Initiator Peroxycarbonsäureester oder Dialkylperoxide verwendet.

6. Verfahren nach einem der Ansprüche 1 bis 5
**dadurch gekennzeichnet,**
daß man den Initiator in einem Verhältnis von 1 bis 50 Molprozent bezüglich des eingesetzten Alkens verwendet.

7. Verfahren nach einem der Ansprüche 1 bis 6,
**dadurch gekennzeichnet,**
daß man die Reaktion bei einer Temperatur von 50 bis 240°C und unter Normaldruck oder erhöhtem Druck durchführt.

8. Verfahren nach einem der Ansprüche 1 bis 7,
**dadurch gekennzeichnet,**
daß man die Reaktion in einem Rohrreaktor unter erhöhtem Druck durchführt.

9. Verfahren nach einem der vorhergehenden Ansprüche,
**dadurch gekennzeichnet,**
daß man nach Durchführung der Reaktion des Produkt aus dem Reaktionsgemisch abtrennt und nicht umgesetzten sekundären Alkohol in das Reaktionsgemisch rückführt.

## Claims

1. Process for the production of monomeric tertiary alcohol addition products by radical addition of a secondary alcohol to an alkene,
**wherein**
the reaction is carried out as a continuous process in the presence of an organic peroxide as a radical initiator, a ratio of 3 to 50 moles secondary alcohol per mole alkene used and with an average contact time of the reaction mixture of up to a maximum of 1 hour.

2. Process as claimed in claim 1,
**wherein**
the reaction is carried out with an average contact time of the reaction mixture of up to a maximum of 30 minutes.

3. Process as claimed in claim 1 or 2,
**wherein**
an alkene with 2 to 20 C atoms is used.

4. Process as claimed in one of the claims 1 to 3,
**wherein**
a secondary alcohol with 3 to 14 C atoms is used.

5. Process as claimed in one of the claims 1 to 4,
**wherein**
peroxycarboxylic acid esters or dialkyl peroxides are used as the radical initiator.

6. Process as claimed in one of the claims 1 to 5,
**wherein**
the initiator is used in a ratio of 1 to 50 mole percent with respect to the alkene used.

7. Process as claimed in one of the claims 1 to 6,
**wherein**
the reaction is carried out at a temperature of 50 to 240°C and under normal pressure or increased pressure.

8. Process as claimed in one of the claims 1 to 7,
**wherein**
the reaction is carried out in a tube reactor under increased pressure.

9. Process as claimed in one of the previous claims,
**wherein**
after carrying out the reaction the product is separated from the reaction mixture and non-reacted secondary alcohol is fed back into the reaction mixture.

## Revendications

1. Procédé de préparation de produits d'addition monomères tertiaires d'alcools par addition radicalaire d'un alcool secondaire sur un alcène, caractérisé en ce qu'on effectue la réaction sous forme d'un procédé continu en présence d'un peroxyde organique en tant qu'amorceur radicalaire, avec un rapport de 3 à 50 moles d'alcool secondaire par mole d'alcène mis en oeuvre et un temps de contact moyen du mélange réactionnel jusqu'à 1 heure au maximum.

2. Procédé selon la revendication 1, caractérisé en ce qu'on effectue la réaction avec un temps de contact moyen du mélange réactionnel jusqu'à 30 minutes au maximum.

3. Procédé selon la revendication 1 ou 2, caractérisé en ce qu'on utilise un alcène avec 2 à 20 atomes.

4. Procédé selon l'une des revendications 1 à 3, caractérisé en ce qu'on utilise un alcool secondaire avec 3 à 14 atomes de C.

5. Procédé selon l'une des revendications 1 à 4, caractérisé en ce qu'on utilise des esters peroxycarboxyliques ou des diakylperoxydes en tant qu'amorceurs radicalaires.

6. Procédé selon l'une des revendications 1 à 5, caractérisé en ce qu'on utilise l'amorceur dans un rapport de 1 à 50 pourcent molaire par rapport à l'alcène mis en oeuvre.

7. Procédé selon l'une des revendications 1 à 6, caractérisé en ce qu'on conduit la réaction à une température de 50 à 240°C et à la pression normale ou à pression accrue.

8. Procédé selon l'une des revendications 1 à 7, caractérisé en ce qu'on effectue la réaction sous pression accrue dans un réacteur tubulaire.

9. Procédé selon l'une des revendications précédentes, caractérisé en ce qu'on sépare le produit du mélange réactionnel après avoir effectué la réaction et que l'on recycle l'alcool secondaire non transformé dans le mélange réactionnel.
